(19) Europäisches Patentamt
European Patent Office
Office européen des brevets

(11) EP 3 562 471 B1

(12) EUROPEAN PATENT SPECIFICATION

(45) Date of publication and mention of the grant of the patent:
**23.06.2021 Bulletin 2021/25**

(21) Application number: **18727391.7**

(22) Date of filing: **18.05.2018**

(51) Int Cl.:
*A61K 8/73* (2006.01) *A61K 8/46* (2006.01)
*A61Q 19/10* (2006.01) *A61K 8/04* (2006.01)

(86) International application number:
**PCT/GB2018/051346**

(87) International publication number:
**WO 2018/211285 (22.11.2018 Gazette 2018/47)**

# (54) HAND PUMP FOAMER COMPRISING A SKIN AND HAND CLEANSING COMPOSITION COMPRISING SCRUBBING MATERIALS

SCHÄUMERPUMPE ENTHALTEND EINE HAUT- UND HANDREINIGUNGSZUSAMMENSETZUNG MIT SCHEUERMATERIALIEN

POMPE À MOUSSE CONTENANT UNE COMPOSITION NETTOYANTE POUR LA PEAU ET LES MAINS COMPRENANT DES MATIÈRES PURIFIANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2017 GB 201708104**

(43) Date of publication of application:
**06.11.2019 Bulletin 2019/45**

(73) Proprietor: **Deb IP Limited**
**Denby, Derbyshire DE5 8JZ (GB)**

(72) Inventors:
- **KAMPS, Nicole**
**Derbyshire**
**DE5 8JZ (GB)**
- **VEEGER, Marcel**
**D-47805 Krefeld (DE)**

(74) Representative: **Elsy, David**
**Withers & Rogers LLP**
**4 More London Riverside**
**London SE1 2AU (GB)**

(56) References cited:
**DE-A1- 10 148 393**
**US-A1- 2012 202 730**
**US-A1- 2013 338 052**

- **Anonymous: "Sodium Laureth Sulfate" In: Joanne Nikitakis, Beth Lange: "International Cosmetic Ingredient Dictionary and Handbook", 2018, Personal Care Products Council- wINCI, XP002782242, ISBN: 1-882621-55-7 abstract**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 562 471 B1

## Description

The invention relates to hand pump foamer apparatus comprising foamable skin and hand cleansing compositions comprising scrubbing materials, and to their use.

Liquid skin and hand cleansing formulations are generally known in the art. They are conventionally provided in containers that are poured or have pumps to pump the liquid compositions onto the skin to be cleaned. Such liquid soaps often have very good cleaning efficiency.

Formulations which are foamed before being placed on the skin or hands are also generally known in the art. Foams tend to be much easier to spread than the corresponding liquid and in addition there is much less waste due to splashing or run-off since the foam has a much higher surface tension than a liquid. One problem associated with foams compared with liquid soaps is they tend to have a lower cleaning efficiency. However, foams have a much higher surface area than un-foamed liquid, so if the cleaning efficiency of the foam can be improved, it is possible to produce foams with the same cleaning power, as obtained with un-foamed liquid but which require much less of the initial liquid to be used.

With the addition of mechanical scrubbers, also known as scrubbing agents, the cleaning performance of liquid soaps can be enhanced. Liquid soaps with suspended scrubbing agents tend to have a viscosity range at their lower end that is higher than that of basic liquid soaps. In order to provide the structure to suspend the mechanical scrubbers of an appropriate size to provide cleaning benefits, these formulations have tended to have a critical strain force (or yield value) superior or equal to 30 dynes/$cm^2$, for example associated with non-Newtonian rheological behaviour. Critical strain force is defined as the stress which must be applied before flow will start, although related to viscosity, it is more dependent on the characteristics of the rheological additive used.

As indicated above, the use of foaming hand cleansers offer many benefits over liquid hand soaps. Typically, such foamable soaps are not capable of suspending mechanical scrubbers of sufficient size to provide cleaning benefits.

WO 2014/019944 describes a foamable gritty foam composition comprising particulate mechanical scrubbers with particles sized from 100 microns to about 800 microns as capable of being foamed through a non-aerosol, or unpressurised foam dispenser. The formulation describes using between 0.5% weight for weight to about 30% weight for weight of a solvent, such as D-limonene or sunflower methyl ester. A wide range of thickeners are described, including those as diverse as xanthan gum, guar gum, quaternised guar gum, alginate, bentonite and fumed silica. One problem associated with such prior art formulations is that they comprise non-aqueous solvents, such as D-limonene, sunflower oil methyl esters or other organic solvents. Such organic solvents are not always acceptable to consumers. Currently there is a need to try and reduce or remove such solvents.

Many prior art formulations use carbomers. Such formulations typically have a pH of 5.5 - 8. The ability to produce a formulation with a pH lower than 5.5, would allow the use of alternative preservatives and produce formulations with improved cleansing properties.

WO 2005/107699 describes an alternative foaming formulation with particles suspended within it that utilises carbomers and a solution of sodium chloride as a preferred system. Such thickeners require a predetermined pH or range of pH to thicken the liquid if pH of the liquid falls outside that range the thickener no longer acts as a thickener.

Other formulations using carboxymethylcellulose (CMC) are known in the art (US 2012/02027304). CMC provides a high viscosity with reduced foaming and instability problems.

The Applicant has identified that it is possible to use xanthan gum, and especially smooth flow xanthan gums, in combination with sulphated castor oils, to produce skin and hand cleansing compositions comprising scrubbing agents.

The invention provides a hand pump foamer apparatus as defined in the claims. Xanthan gums are polysaccharides produced by microorganisms of the genus *Xanthomonas,* and in particular *Xanthomonas campestris.*

The gum is typically obtained by fermentation of the microorganism and is generally used for a wide range of different uses in the food and chemical industries. Thickening compositions using xanthan gum tend to have a non-uniform or "chunky" flow characteristic, typical to that found with tomato ketchup. Whilst such conventional xanthan gum may be used, the Applicant has unexpectedly found that so-called "smooth-flow" xanthan gums produce better foamable compositions because they have lower viscosity than standard xanthan gums. Such smooth-flow xanthan gums are described in, for example, EP 0005030 and US 4,263,399. Such smooth-flow xanthan gums are available from CP Kelco, under the name of, for example, Keltrol®CG-SFT. Such smooth-flow xanthan gums are typically formed and made using high shear conditions that produces a material that is generally homogeneous, compared with native uneven xanthan gums. The material also has lower extensional viscosity than conventional xanthan gum and is stable under high shear conditions compared to conventional xanthan gum which shows reduced viscosity on being subjected to shearing.

Typically, additional thickeners such as cellulose gums are not used. The addition of cellulose gums, for example, was found to reduce foamability.

Typically, the composition has a viscosity of 500 to 5000 mPa.s. More typically this is less than 3500 mPa.s, between 800 - 2000 mPa.s, especially 800 - 1300 mPa.s. Above 3500 mPa.s or above 2000 mPa.s produces reduced foaming.

Typically the composition has a yield value of at least $0.0003N/cm^2$ up to typically $0.002N/cm^2$, more typically 0.0005 to 0.001 $N/cm^2$ or $0.0008N/cm^2$ (30 $dynes/cm^2$ up to typically 200 $dynes/cm^2$, more typically 50 to 100 $dynes/cm^2$ or 80 $dynes/cm^2$).

The pH of the composition may be below 6, especially 5.5 or below, more typically above pH 3, or above pH 4.

Sulphated (also known as sulphonated) castor oil is castor oil that has been treated with, for example, sulphuric acid to produce an anionic surfactant.

Typically, the composition comprises 1 to 8% by weight, 1 to 5.5, more typically 3 to 5% or 3 to 3.5% by weight of sulphated castor oil. Sulphated castor oil acts as a surfactant and improves the stability of the formula. The sulphated castor oil may be provided as substantially pure material or containing, for example, 70% castor oil pure sulphated castor oil.

Typically 0.1 to 1, 0.2 to 0.9 more typically 0.4 to 0.8% by weight of xanthan gum may be used.

"Conventional" xanthan gums are those which have not been made using higher shear conditions, for example as described above. Application of high shear rates to such gums reduces the viscosity of such gums.

Both conventional xanthan gums and smooth flow xanthan gums, or mixtures thereof may be used.

However, where conventional xanthan gums, the Applicant identified that higher levels of xanthan gum produce higher viscosities which inhibit foam formation.

Accordingly, the amount of xanthan gum, for example conventional xanthan gum (but alternatively smooth xanthan gum or mixtures thereof) may be limited to 0.1 - 0.5%, more typically 0.35 - 0.45% by weight. Typically, this reduces the viscosity to below 4000 mPa.s.

Typically less than 5%, less than 3% or less than 2% by weight of at least one non-aqueous solvent such an organic solvent is used, more typically no non-aqueous solvent is used. Non-aqueous solvent may be one or both of D-limonine and sunflower oil methyl ester. Other solvents include glycol ethers, esters, alcohols, other terpenes and aromatic-free white spirit. Non aqueous solvents such as D-limonine may be omitted from the formulation.

The composition typically comprises 2 to 30% by weight, more typically 2 to 20%, or 3 to 10% by weight of the additional surfactant (component d)).

The surfactant is selected from:

i) Amphoteric Surfactants

The amphoteric surfactant may be any one or combination of betaines, acyl ethylene diamines, amino-acids derivatives, imidazolines. Alternatively, the amphoteric surfactant may be any one or combination of acylamphoacetate, acylamphodiacetate, acylamphodipropionate, sodium cocoglycinate, sodium alkyliminodipropionate, cocamidopropyl betaine, sodium cocoamphoacetate.

The betaine may be any one or combination of coco betaine and cocamidopropyl betaine.

ii) Non-Ionic Surfactants

The non-ionic surfactant may be any one or combination of glucosides, ethoxylated fatty alcohols, ethoxylated fatty acids, saccharose esters, sorbitan esters, alkanolamides, glycerol alkyl esters, polyoxyethylene glycol alkylphenol esters.

iii) Anionic Surfactants

The anionic surfactant may be any one or combination of lauryl sulphates, lauryl ether sulphates, sulphosuccinates, carboxylates (i.e. sodium oleate), carboxylic acid esters (i.e. sodium dilaureth citrate), alkyl sulfate (i.e. sodium lauryl ether sulfate, ammonium alkyl sulfate, alkyl and alkyl-aryl sulfonates (i.e. sodium dodecyl benzene sulfonate), sulfosuccinates (i.e. disodium lauryl ether sulfosuccinate), isethionates (i.e. sodium cocoyl isethionate, ammonium cocoyl isethionate), taurates (i.e. sodium methyl cocoyl taurate, sodium methyl oleoyl taurate)acyl glutamates (i.e. sodium lauroyl glutamate, sodium cocoyl glutamate, disodium cocoyl glutamate), sarcosinate (i.e. cocoyl sarcosinate), alkylpolyglucosides (i.e. decyl glucoside, sodium lauryl glucose carboxylate, caprylyl/capryl glucoside).
or

iv) one or more fatty acid glucamides,

The fatty acid glucamide typically has the general formula:

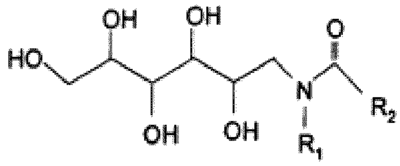

(1)

where:

$R^1$ = H or a C1 or C4 alkyl, most typically H or $-CH_3$, especially $-CH_3$

$R^2$ = $C_6$ to $C_{20}$ linear or branched hydrocarbon residue, most typically $C_8$ to $C_{10}$ linear or unbranched hydrocarbon residue. The hydrocarbon residue may be saturated or unsaturated.

Mixtures of the glucoamines may be used. For example, the glucamide may be a mixture of capryloyl/caproyl methyl glucamide; lauroyl/myristoyl methyl glucamide; or cocoyl methyl glucamide. Mixtures of these may also be used, for example, a mixture of cocoyl methyl glucamide and capryloyl/caproyl methyl glucamide may be used. Cocoyl methyl glucamide comprises fatty acid residues from coconut oil.

The surfactant may be sodium laureth sulfate or disodium laureth sulfosuccinate. Mixtures of one or more fatty acid glucamides, sodium laureth sulfate (SLS) and disodium laureth sulfosuccinate (DLS) may be used, or indeed mixtures of other surfactants. Capryloyl/caproyl methyl glucamide optionally mixed with sodium laureth sulfate and/or disodium laureth sulfosuccinate may be used.

The amount of fatty acid glucamide may be between 2.0 and 5.5% by weight, especially 2.5 to 3.5% by weight. SLS may be present in 3.0 to 8.0% by weight. DLS may be present between 2.0 and 4.5% by weight.

Typically the composition may comprise 0.5 to 2.5% by weight, more typically 1.0 to 2.0% of emulsifier. The emulsifier is selected from PEG-18, castor dioleate, polyglycerin 4-caproate, d-n-octylether lauryl glucoside, caprylyl glycol, sorbitan sequicaprate, hydrogenated castor oil, PEG-10 glyceryl oleate and gemini surfactants and mixtures thereof. Gemini surfactants are a family of surfactant molecules possessing more than one hydrophobic tail and hydrophilic head group. Gemini surfactants usually have better surface-active properties than corresponding conventional surfactants of equal chain length. For example, they have the hydrophobic to hydrophilic head groups connected to one another via a spacer. These are described in, for example, a review article by S. K. Hate and S.P. Moulik, Current Science, Vol 82(9) (2002) 1101-1111. Typically the emulsifier is glyceryl oleate citrate, (commercially available as Dermofeel® Easymuls Plus, Dr Straetmans GmbH), or polyglyceryl 3 caprate (commercially available under the name Tegosoft® PC 31, Evonik Industries AG).

The composition may comprise 5 to 15% by weight of scrubbing agent. The scrubbing agent is particulate and is any one or combination of a vegetable based scrubbing agent, a synthetic based scrubbing agent and a mineral based scrubbing agent, and the particles may have a size in a range from about 100 microns to about 800 microns, or in a range from about, or in a range from about 200 to about 700, or in a range from about 300 to about 500 microns. The particles may have any size in this range, or be in any narrow range than 100 to 800 microns, or there may be a mixture of any size of particles in this broad range present in the composition. The vegetable based scrubbing agent may be any one or combination of cornmeal, olive stone, walnut shells, ground fruit stones, ground corn meal, ground fruit shells. The synthetic based scrubbing agent may be any one or combination of polyethylene and polypropylene. The mineral based scrubbing agent may be any one or combination of ground shellfish, pumice and silica.

The composition may comprise 0.002 to 2% by weight of at least one preservative. The preservative may be an organic acid, such as sodium benzoate or potassium sorbate. The preservative may be present in an amount 0.1 to 2.0% or 0.1 to 1.5%, or 0.1 - 0.9% or 0.5 - 0.9% by weight.

pH modifiers may be used to adjust the pH. These include organic acids such as citric acid and lactic acid.

Typically, less than 0.5%, less than 0.4%, less than 0.2%, less than 0.1% wt or no additional thickeners are included. Such additional thickeners include polysaccharides such as carboxymethylcellulose (CMC). Typically, no additional thickener, such as CMC, in incorporated.

The formulation may comprise 0 to 10% by weight of auxiliaries and/or other additives, these include, for example, colourants, salts (such as sodium chloride or monosodium phosphate), oleic acid, pearlessing agents and, fragrances. Typically these are present in an amount of 1 to 9%, 3 - 7% or 5% by weight.

Water is added to make the total percentage weight 100%.

The invention provides hand pumping apparatus comprising compositions of the invention. Such hand pumping apparatus include those described for example, in WO 2005/107699. Typically the apparatus uses a hand pumping dispenser, rather than a pre-pressurised dispenser.

Methods of washing skin using the compositions of the invention are also provided. This typically includes expelling the composition through the foaming apparatus to produce a foam, and applying the foam to a portion of the

skin of the subject. This may then be washed off using water, together with at least a portion of any dirt found on the skin. Skin is typically the hands and/or the face of a subject.

The invention will now be described by way of example with reference to the following table. The table shows examples of different formulae and effect of different additives on cleansing foamability and stability of the foam.

Cleansing performance was typically carried out using the following method:

**Foam Production**

The formulations described below were tested by evaluating the Foam quality as a ratio between the g product / ml foam. This was aimed to be typically 1:4 or even better 1:5 but not lower or equal to 1:2.

**Cleansing Performance**

Cleansing performance was typically carried out using the following method:
The test model of the hand washing test with standardised soiling or paint gives information about the cleaning effect of the products to be tested. For relevance in practice, it is necessary that all subjects have a characteristic skin structure on the palms of the hands caused by manual work. Using one product at time, the following test is carried out in the morning and afternoon:

<u>Test Procedure With Water:</u>

0.5g of soiling (model soiling, practice soiling or paint) is distributed on the palm and the back of the hand and rubbed in.

Rub in and leave to dry within 2 min.

1.2 g of cleansing composition are applied and rubbed in
1 ml of water is added twice within 1 min.

Rinse under hand warm running tap water and remove as much or all of the soil as possible within 30 s

Visual assessment of the residual soiling (RS) on the back of the hand and the palm according to the scale, see below.

<u>Test Procedure Without Water:</u>

0.5 g of soiling (model soiling, practice soiling or paint) are distributed on the palm and on the back of the hand and rubbed in

Leave to dry for 1 1/2 min
1.2 g of cleaning composition are applied and rubbed in
Using a cellulose paper, the soiling on the hand surfaces is removed together with the product

Visual assessment of the residual soiling (RS) on the back of the hand and the palm according to the scale, see below.
0=clean 5=no cleaning effect (graduation in steps of 0.5 possible).
The percentage cleaning effect is calculated according to the following formula:

$$\text{cleaning effect } [\%] = \frac{10 - (\overline{RS}_{palm} + \overline{RS}_{back})}{10} * 100\%$$

$RS_{palm}$=mean value of the residual soiling on palms of n measurement series (subjects). $RS_{back}$=mean value of the residual soiling on backs of hands of n measurement series (subjects).

Since the deterioration of the cleaning effect has a broader variation range, an absolute deviation of 5% between two measurement series is allowable.

Composition of a suitable model soiling:

| | |
|---|---|
| Flame soot: | 5.42% |
| Iron oxide ($F_{e2}O_3$): | 0.72% |

In the Examples below the amount of sulphonated castor oil used is the amount of 100% pure sulphonated castor oil. Examples are wt %.

Keltrol is "smooth" xanthan gum. Jungbunzlauer "conventional" xanthan gum.

Typically water was added to a vessel and mixed with the xanthan gum by stirring. Scrubbing agents and preservatives were then added followed by the surfactants; including the sulphonated castor oil, then the emollients and perfume. The pH was adjusted using the pH modifiers.

A list of components used in the examples by trade name and INCI name follows, along with examples of the invention and comparative examples.

The following examples provide particularly good hand cleansing, foamability and stability: B7 to B16, C1 to D6, D8 to D10, E2 to F3, O1 and O1.1 to 01.3.

| Trade Name | INCI Name |
|---|---|
| Water | Aqua |
| **Yield improvement** | |
| Keltrol CG-SFT | Xanthan Gum |
| Sucraclear HC-31 | Cellulose Gum, Chondrus Crispus (Carrageenan) Extract, Ceratonia Siliqua Gum, Sucrose |
| Jungbunzlauer FEDCS-PC | Xanthan Gum |
| Simulgel FL | Hydroxyethyl Acrylate / Sodium Acryloyldimethyl Taurate Copolymer; Isohexadecane; Polysorbate 60 |
| Exilva | |
| Aristoflex TAC | Ammonium Acryloyldimethyltaurate/ Carboxyethyl Acrylate Crosspolymer |
| Actigum VSX 20 | sclerotium gum and xanthan gum |
| Kelcogel CG-LA | Gellan gum |
| Rhodicare S | Xanthan Gum |
| Walocel CRT 50.000 PA 07 | Cellulose Gum |
| **Nonionics** | |
| Gluco Tain Clear | Capryloyl/Caproyl Methyl Glucamide |
| Gluco Tain Care | Cocoyl Methyl Glucamide |
| Rewopal LA 6 | Laureth 6 |
| Marlipal 24/70 | Trideceth 7 |
| Mackamine LA | Lauramine Oxide |
| Bio Saponins | Saponins |
| Steposol MET 10 U | N,N-dimethyl 9-decanamide |
| Betaine Coco Base | Cocoamidopropyl Betaine |
| **Anionics** | |
| SLES | Sodium Laureth Sulfate |
| Sulfated Castor Oil 75 WS | Sulfated Castor Oil |
| Rewopol SBF A 30 B | Disodium Laureth Sulfosuccinate |
| Protelan LS9011 | Natrium-N-lauroylsarkosinat |
| Greenbentin MLS/100 | Fatty acids C12-16 and C18-unsaturated, esters with polyethylene glycol mono-Me ether |
| Eucarol Age SS | Disodium alkylpolyglucose |
| TEA Laurylsulfat | TEA-Lauryl Sulphate |
| **Emulsifieres/Stabiliser** | |
| Dermofeel Easymuls Plus | Glyceryl Oleate Citrate |
| Dermosoft Octiol | Caprylyl Glycol |
| Versatil MPC | Methylpropanediol; Phenoxyethanol; Caprylyl Glycol |
| Antilsoft SC | Sorbitan Sesquicaprylate |
| Oxypon 401 | PEG-9 Cocoglycerides |
| Glycerin pflanzlich 99,5% | Glycerin |
| Polyglycerin-6 | Glycerin |
| Cetiol OE | di-n-octyl Ether |
| Glucopon 650 EC/HH | Lauryl Glucoside |
| Cosmacol ELI | C12-13 Alkyl Lactate |
| Tegosoft PC 31 | Polyglyceryl 3 Carprate |
| **Preservative** | |
| Organic acid | Sodium Benzoate |
| Organic acid | Potassium Sorbate |
| **Scrubber** | |
| Cornmeal | Corn (Zea Mays) Meal |
| Olive Stone Granules | Olea Europaea (olive) Seed Powder |
| **Auxillaries** | |
| Fragrance | Parfum (Fragrance) |
| Palmera A1813 | Oleic Acid |
| pH Adjuster | Citric acid |
| Mononatriumphosphat calc. | Mononatriumphosphat calc. |
| Sodiumchlorid solution 15% | Sodium Chloride |
| **Performance** | |
| Cleansing effect (Method as described) as requested **> 70%** | |
| Foamability - Foamquality (Methode as described) as requested **> 3,9** | |
| Stability (Method as described) as requested **min +** | |

| Trade Name | A1 | B1 | B2 | B3 | B4 | B5 | B6 | B7 | B8 | B9 | B10 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.10 | 0.30 | 0.30 | 0.30 | 0.30 | 0.30 | 0.30 | 0.30 | 0.30 | 0.30 | 0.30 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | | | | | | | 2.20 | 3.30 | 3.30 | 3.30 | 3.30 |
| Gluco Tain Care | | | | | | | | | | | |
| Rewopal LA 6 | 6.00 | | | | | | 4.00 | | | | |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 3.64 | 3.38 | 3.64 | 6.50 | 10.40 | 13.00 | 6.50 | 3.37 | 3.38 | 5.20 | 5.20 |
| Sulfated Castor Oil 75 WS | 3.00 | 3.00 | 3.00 | 3.00 | 3.00 | 3.00 | 3.00 | 3.00 | 3.00 | 2.00 | 3.00 |
| Rewopol SBF A 30 B | 2.10 | 1.20 | 2.10 | 12.60 | 3.50 | 3.50 | | | 1.75 | | |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | 1.50 | 1.50 | 1.50 | 1.50 | 1.50 | 1.50 | 1.50 | 1.50 | 1.50 | 1.50 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 95% | 65% | 65% | 65% | 65% | 65% | 95% | 71% | 71% | 71% | 71% |
| Foamability | 3.0 | 4.0 | 4.0 | 4.0 | 4.0 | 4.0 | 3.0 | 4.5 | 4.5 | 4.5 | 4.5 |
| Stability | + | + | + | + | + | + | + | + | + | + | + |

| Trade Name | B11 | B12 | B13 | B14 | B15 | B16 | B17 | B18 | B19 | B20 | B21 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.30 | 0.30 | 0.30 | 0.30 | 0.30 | 0.30 | 0.30 | 0.30 | 0.30 | 0.30 | 0.30 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | 3.30 | 3.30 | 3.30 | 3.30 | 3.30 | | | | | | |
| Gluco Tain Care | | | | | | 2.88 | 1.92 | | | | |
| Rewopal LA 6 | | | | | | | 4.00 | 4.00 | 4.00 | 4.00 | 4.00 |
| Marlipal 24/70 | | | | | | | | | | | |
| Mackamine LA | | | | | | | | | | | |
| Bio Saponins | | | | | | | | | | | 4.00 |
| Steposol MET 10 U | | | | | | | | | | | |
| Betaine Coco Base | | | | | | | | | | | |
| **Anionics** | | | | | | | | | | | |
| SLES | 5.20 | 5.20 | 5.20 | 5.20 | 5.20 | 3.38 | 6.50 | 4.68 | 6.50 | 6.50 | 6.50 |
| Sulfated Castor Oil 75 WS | 3.00 | 3.00 | 3.00 | 3.00 | 4.00 | 3.00 | 3.00 | 3.00 | 3.00 | 3.00 | 3.00 |
| Rewopol SBF A 30 B | | | | | | | | 2.10 | 2.10 | | |
| Protelan LS9011 | | | | | | | | | | 1.80 | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | 1.00 | 2.00 | 1.50 | 1.50 | 1.50 | 1.50 | 1.50 | 1.50 | 1.50 | 1.50 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.00 | 2.00 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 71% | 71% | 71% | 71% | 71% | 71% | 95% | 85% | 85% | 85% | 90% |
| Foamability | 4.5 | 4.5 | 4.5 | 4.5 | 4.5 | 4.5 | 3.0 | 3.0 | 3.0 | 3.0 | 3.0 |
| Stability | + | + | + | + | + | + | + | + | + | - | - |

| Trade Name | B22 | B23 | B24 | B25 | B26 | B27 | B28 | B29 | B30 | B31 | B32 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.30 | 0.30 | 0.30 | 0.30 | 0.30 | 0.30 | 0.30 | 0.30 | 0.30 | 0.30 | 0.30 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | | | | | | | | | | | |
| Gluco Tain Care | | | | | | | | | | | |
| Rewopal LA 6 | 4.00 | 4.00 | 6.00 | 6.00 | 6.00 | 6.00 | 6.00 | 6.00 | 6.00 | 6.00 | 6.00 |
| Marlipal 24/70 | | | | | | | | | | | |
| Mackamine LA | | 6.00 | 4.00 | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 6.40 | 6.50 | 6.50 | 3.64 | 3.6 | 3.64 | 3.64 | 3.6 | 3.64 | 3.64 | 3.64 |
| Sulfated Castor Oil 75 WS | 3.00 | 3.00 | 3.00 | 3.00 | 3.00 | 3.00 | 3.00 | 3.00 | 3.00 | 3.00 | 3.00 |
| Rewopol SBF A 30 B | | | | 2.10 | 2.10 | 2.10 | 2.10 | 2.10 | 2.10 | 2.10 | 2.10 |
| Protelan LS9011 | | | | | | | | | | | |
| Greenbentin MLS/100 | | | | | | | | | | | |
| Eucarol Age SS | 1.80 | | | | | | | | | | |
| TEA Laurylsulfat | | | | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | 1.00 | 1.00 | 1.50 | 2.00 |
| Glucopon 650 EC/HH | | | | | | | | | | | |
| Cosmacol ELI | | | | | | | | | | | |
| Tegosoft PC 31 | 1.50 | 1.50 | 1.50 | 1.50 | 1.50 | 1.50 | 1.50 | 1.50 | 1.50 | 1.50 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.00 | 0.50 | | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 95% | 95% | 95% | 95% | 95% | 95% | 95% | 95% | 95% | 95% | 95% |
| Foamability | 3.0 | 3.0 | 3.0 | 3.0 | 3.0 | 3.0 | 3.0 | 3.0 | 3.0 | 3.0 | 3.0 |
| Stability | - | - | - | + | - | - | - | + | + | + | + |

| Trade Name | B33 | B34 | B35 | B36 | B37 | B38 | B39 | B40 | B41 | B42 | B43 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.30 | 0.30 | 0.30 | 0.30 | 0.30 | 0.30 | 0.30 | 0.30 | 0.30 | 0.30 | 0.30 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | | | | | | | | | | | |
| Gluco Tain Care | | | | | | | | | | | |
| Rewopal LA 6 | 6.00 | 6.00 | 6.00 | 6.00 | 6.00 | 6.00 | 6.00 | 6.00 | 6.00 | 6.00 | 6.00 |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 3.6 | 3.64 | 3.6 | 3.64 | 3.64 | 3.64 | 3.64 | 3.64 | 3.64 | 3.64 | 3.64 |
| Sulfated Castor Oil 75 WS | 3.00 | 3.00 | 3.00 | 3.00 | 3.00 | 3.00 | 3.00 | 3.00 | 3.00 | 3.00 | 3.00 |
| Rewopol SBF A 30 B | 2.42 | 2.38 | 2.35 | 2.35 | 2.31 | 2.28 | 2.10 | 2.10 | 2.10 | 2.10 | 2.10 |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | | | | | 1.50 | | | 1.00 | 0.70 |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | 1.50 | | | |
| Polyglycerin-6 | | | | | | | | | 1.50 | | |
| Cetiol OE | | | | | | | | | | | |
| Glucopon 650 EC/HH | | | | | | | | | | | |
| Cosmacol ELI | | | | | | | | | | | |
| Tegosoft PC 31 | 0.60 | 0.70 | 0.80 | 0.80 | 0.90 | 1.00 | | | | | |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 95% | 95% | 95% | 95% | 95% | 95% | 95% | 95% | 95% | 95% | 95% |
| Foamability | 3.0 | 3.0 | 3.0 | 3.0 | 3.0 | 3.0 | 3.0 | 3.0 | 3.0 | 3.0 | 3.0 |
| Stability | - | - | - | - | - | - | + | - | - | + | + |

| Trade Name | B44 | B45 | B46 | B47 | B48 | B49 | B50 | B51 | B52 | B53 | B54 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.30 | 0.30 | 0.30 | 0.30 | 0.30 | 0.30 | 0.30 | 0.30 | 0.30 | 0.30 | 0.30 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | | | | | | | | | | | |
| Gluco Tain Care | | | | | | | | | | | |
| Rewopal LA 6 | 6.00 | 6.00 | 6.00 | 6.00 | 6.00 | 6.00 | 6.00 | 6.00 | 6.00 | 6.00 | 6.00 |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 3.64 | 3.64 | 3.64 | 3.64 | 3.64 | 3.64 | 3.64 | 3.64 | 3.64 | 3.64 | 3.64 |
| Sulfated Castor Oil 75 WS | 3.00 | 3.00 | 3.00 | 3.00 | 3.00 | 3.00 | 3.00 | 3.00 | 3.00 | 3.00 | 3.00 |
| Rewopol SBF A 30 B | 2.10 | 2.10 | 2.10 | 2.10 | 2.10 | 2.10 | 2.10 | 2.10 | 2.10 | 2.10 | 2.10 |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | 0.50 | 0.40 | 0.30 | 0.20 | | | | | | | |
| Dermosoft Octiol | | | | | 0.50 | 1.00 | 1.50 | | | | |
| Versatil MPC | | | | | | | | 0.50 | 1.00 | 1.50 | |
| Antilsoft SC | | | | | | | | | | | 1.00 |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | | | | | 1.50 | 1.50 | 1.50 | 1.50 | 1.50 | 1.50 | |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 95% | 95% | 95% | 95% | 95% | 95% | 95% | 95% | 95% | 95% | 95% |
| Foamability | 3.0 | 3.0 | 3.0 | 3.0 | 3.0 | 3.0 | 3.0 | 3.0 | 3.0 | 3.0 | 3.0 |
| Stability | - | - | - | - | + | + | + | + | + | + | + |

| Trade Name | B55 | B56 | B57 | B58 | B59 | B60 | B61 | B62 | B63 | C1 | C2 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.30 | 0.30 | 0.30 | 0.30 | 0.30 | 0.30 | 0.30 | 0.30 | 0.30 | 0.40 | 0.40 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | | | | | | | | | | 3.30 | 3.30 |
| Gluco Tain Care | | | | | | | | | | | |
| Rewopal LA 6 | 6.00 | 6.00 | 6.00 | 6.00 | 6.00 | | | | | | |
| Marlipal 24/70 | | | | | | 6.00 | 6.00 | 6.00 | 6.00 | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 3.64 | 3.64 | 3.64 | 3.64 | 3.64 | 3.64 | 3.64 | 3.64 | 3.64 | 3.38 | 5.20 |
| Sulfated Castor Oil 75 WS | 3.00 | 3.00 | 3.00 | 3.00 | 3.00 | 3.00 | 3.00 | 3.00 | 3.00 | 3.00 | 3.00 |
| Rewopol SBF A 30 B | 2.10 | 2.10 | 2.10 | 2.10 | 2.10 | 2.10 | 2.28 | 2.45 | 2.63 | 1.75 | |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | 0.80 | 0.50 | | | | | | | | | |
| Oxypon 401 | | | 1.50 | 2.25 | 3.00 | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | | | | | | 1.50 | 1.00 | 0.50 | | 1.00 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 95% | 95% | 95% | 95% | 95% | 85% | 95% | 95% | 95% | 71% | 72% |
| Foamability | 3.0 | 3.0 | 3.0 | 3.0 | 3.0 | 3.0 | 3.0 | 3.0 | 3.0 | 4.5 | 4.5 |
| Stability | - | - | - | - | - | - | + | + | - | + | + |

| **Trade Name** | **C3** | **C4** | **C5** | **C6** | **C7** | **C8** | **D1** | **D2** | **D3** | **D4** | **D5** |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.40 | 0.40 | 0.40 | 0.40 | 0.40 | 0.40 | 0.50 | 0.50 | 0.50 | 0.50 | 0.50 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | 3.30 | 3.30 | 3.30 | 3.30 | 3.30 | 3.30 | 2.20 | 2.20 | 2.20 | 2.75 | 2.75 |
| Gluco Tain Care | | | | | | | | | | | |
| Rewopal LA 6 | | | | | | | | | | | |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | 1.86 | 1.86 |
| **Anionics** | | | | | | | | | | | |
| SLES | 3.38 | 3.38 | 3.38 | 3.38 | 3.38 | 3.38 | 3.90 | 3.90 | 3.38 | 5.20 | 3.38 |
| Sulfated Castor Oil 75 WS | 3.00 | 2.00 | 4.00 | 3.00 | 3.00 | 3.00 | 3.00 | 2.00 | 2.00 | 3.00 | 3.00 |
| Rewopol SBF A 30 B | 1.75 | 1.75 | 1.75 | 1.75 | 1.75 | 1.75 | 1.75 | 2.45 | 3.15 | | |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | 1.50 | 1.50 | 1.50 | 1.50 | 1.50 | 2.00 | 1.50 | 1.50 | 1.00 | 1.50 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.00 | 2.00 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.50 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 71% | 72% | 73% | 73% | 73% | 73% | 71% | 72% | 74% | 75% | 75% |
| Foamability | 4.5 | 4.5 | 4.5 | 4.5 | 4.5 | 4.5 | 4.5 | 4.5 | 4.5 | 4.5 | 4.5 |
| Stability | + | + | + | + | + | + | + | + | + | + | + |

| Trade Name | D6 | D7 | D8 | D9 | D10 | D11 | D12 | D13 | D14 | D15 | D16 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.50 | 0.50 | 0.50 | 0.50 | 0.50 | 0.50 | 0.50 | 0.50 | 0.50 | 0.50 | 0.50 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | 2.75 | 3.30 | 3.30 | 3.30 | 3.30 | | | | | | |
| Gluco Tain Care | | | | | | | | | | | |
| Rewopal LA 6 | | | | | | 6.00 | 6.00 | 6.00 | 6.00 | 6.00 | 6.00 |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | 1.86 | | | | | | | | | | |
| **Anionics** | | | | | | | | | | | |
| SLES | 7.80 | 3.38 | 3.64 | 5.20 | 3.64 | 3.64 | 3.64 | 3.64 | 3.64 | 3.64 | 3.64 |
| Sulfated Castor Oil 75 WS | 3.00 | 3.00 | 3.00 | 3.00 | 3.00 | 3.00 | 3.00 | 3.00 | 3.00 | 3.00 | 3.00 |
| Rewopol SBF A 30 B | | 1.75 | 1.75 | | 1.75 | 2.10 | 2.10 | 2.35 | 2.31 | 2.28 | 2.45 |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | 1.50 | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | 1.50 | | | | | | | | | |
| Tegosoft PC 31 | 1.50 | | | 1.50 | 1.50 | 1.50 | 1.50 | 0.80 | 0.90 | 1.00 | 0.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 75% | 74% | 73% | 72% | 71% | 95% | 95% | 95% | 95% | 95% | 95% |
| Foamability | 4.5 | 4.5 | 5.0 | 5.0 | 4.5 | 3.0 | 3.0 | 3.0 | 3.0 | 3.0 | 3.0 |
| Stability | + | - | + | + | + | + | + | - | - | - | - |

| Trade Name | D17 | D18 | D19 | D20 | D21 | D22 | D23 | D24 | E1 | E2 | F1 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.50 | 0.50 | 0.50 | 0.50 | 0.50 | 0.50 | 0.50 | 0.50 | 0.60 | 0.60 | 0.80 |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | | | | | | | | | | | |
| Actigum VSX 20 | | | | | | | | | | | |
| Kelcogel CG-LA | | | | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | | | | | | | | | 3.30 | 3.30 | 3.30 |
| Gluco Tain Care | | | | | | | | | | | |
| Rewopal LA 6 | 6.00 | 6.00 | 6.00 | | | | | | | | |
| Marlipal 24/70 | | | | | | | | | | | |
| Mackamine LA | | | | 10.00 | | | | | | | |
| Bio Saponins | | | | | | | | | | | |
| Steposol MET 10 U | | | | | | 4.00 | | | | | |
| Betaine Coco Base | | | 1.86 | 1.86 | 1.86 | 1.86 | 3.10 | | | | |
| **Anionics** | | | | | | | | | | | |
| SLES | 3.64 | 3.64 | 3.64 | 6.40 | 6.50 | 6.50 | 6.40 | 6.40 | 5.20 | 3.38 | 3.38 |
| Sulfated Castor Oil 75 WS | 3.00 | 3.00 | | 3.00 | 3.00 | 3.00 | 3.00 | 3.00 | 3.00 | 3.00 | 3.00 |
| Rewopol SBF A 30 B | 2.42 | 2.38 | | | | | | 3.50 | | 1.75 | |
| Protelan LS9011 | | | | | | | | | | | |
| Greenbentin MLS/100 | | | | | 4.00 | | | | | | |
| Eucarol Age SS | | | | | | | | | | | |
| TEA Laurylsulfat | | | 2.40 | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | 0.60 | 0.70 | 1.50 | 1.50 | 1.50 | 1.50 | 1.50 | 1.50 | 1.50 | 1.50 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | | | | |
| Sodiumchlorid solution 15% | | | | | | | | | | | |
| **Performance** | | | | | | | | | | | |
| Cleansing effect | 95% | 95% | 95% | 65% | 65% | 65% | 65% | 65% | 72% | 74% | 75% |
| Foamability | 3.0 | 3.0 | 3.0 | 3.0 | 3.0 | 3.0 | 3.0 | 3.0 | 3.0 | 4.0 | 4.0 |
| Stability | - | - | - | - | - | - | - | - | + | + | + |

| **Trade Name** | **F2** | **F3** | **G1** | **H1** | **I1** | **J1** | **K1** | **L1** | **L2** | **L3** | **L4** |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | 0.80 | 0.80 | 0.04 | | | | | | | | |
| Sucraclear HC-31 | | | | 0.30 | | | | | | | |
| Jungbunzlauer FEDCS-PC | | | | | 0.30 | | | | | | |
| Simulgel FL | | | | | | 2.00 | | | | | |
| Exilva | | | | | | | 3.00 | | | | |
| Aristoflex TAC | | | | | | | | 0.50 | 0.60 | 0.70 | 1.00 |
| Actigum VSX 20 | | | | | | | | | | | |
| Kelcogel CG-LA | | | 0.08 | | | | | | | | |
| Rhodicare S | | | | | | | | | | | |
| Walocel CRT 50.000 PA 07 | | | | | | | | | | | |
| **Nonionics** | | | | | | | | | | | |
| Gluco Tain Clear | 3.30 | 3.30 | 3.30 | | | | | | | | |
| Gluco Tain Care | | | | | | | | | | | |
| Rewopal LA 6 | | | | 6.00 | 6.00 | 6.00 | 6.00 | 6.00 | 6.00 | 6.00 | 6.00 |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 3.38 | 3.38 | 3.38 | 3.64 | 3.64 | 3.64 | 3.64 | 3.64 | 3.64 | 3.64 | 3.64 |
| Sulfated Castor Oil 75 WS | 3.00 | 3.00 | 3.00 | 3.00 | 3.00 | 3.00 | 3.00 | 3.00 | 3.00 | 3.00 | 3.00 |
| Rewopol SBF A 30 B | 1.75 | | | 2.10 | 2.10 | 2.10 | 2.10 | 2.10 | 2.10 | 2.10 | 2.10 |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | | | | |
| **Emulsifieres/Stabiliser** | | | | | | | | | | | |
| Dermofeel Easymuls Plus | | | | | | | | | | | |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | 8.00 | | | | | | | | | |
| Cosmacol ELI | | | | | | | | | | | |
| Tegosoft PC 31 | 1.50 | 1.50 | 1.50 | 1.50 | 1.50 | 1.50 | 1.50 | 1.50 | 1.50 | 1.50 | 1.50 |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | 1.50 | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | 0.10 | | | | | | | | |
| Sodiumchlorid solution 15% | | | 0.70 | | | | | | | | |
| **Performance** | | | | | | | | | | | |
| Cleansing effect | 74% | 75% | 75% | 95% | 95% | 95% | 95% | 95% | 95% | 95% | 95% |
| Foamability | 4.0 | 4.0 | 3.0 | 3.0 | 3.0 | 3.0 | 3.0 | 3.0 | 3.0 | 3.0 | 3.0 |
| Stability | + | + | - | + | + | + | - | - | - | - | + |

| Trade Name | L5 | M1 | M2 | M3 | M4 | M5 | M6 | N1 | O1 | O2 | O3 |
|---|---|---|---|---|---|---|---|---|---|---|---|
| Water | ad 100 | ad 100 | ad 100 | ad 100 | ad 100 | ad 100 | ad 100 | ad 100 | ad 100 | ad 100 | ad 100 |
| **Yield improvement** | | | | | | | | | | | |
| Keltrol CG-SFT | | | | | | | | | | | |
| Sucraclear HC-31 | | | | | | | | | | | |
| Jungbunzlauer FEDCS-PC | | | | | | | | | | | |
| Simulgel FL | | | | | | | | | | | |
| Exilva | | | | | | | | | | | |
| Aristoflex TAC | 1.00 | | | | | | | | | | |
| Actigum VSX 20 | | 0.10 | 0.20 | 0.30 | 0.40 | 0.50 | 0.60 | | | | |
| Kelcogel CG-LA | | | | | | | | 0.10 | | | |
| Rhodicare S | | | | | | | | | 0.50 | | 0.50 |
| Walocel CRT 50.000 PA 07 | | | | | | | | | | 0.50 | 0.50 |
| **Nonionics** | | | | | | | | | | | |
| Gluco Tain Clear | | | | | | | | | 3.3 | 3.3 | 3.3 |
| Gluco Tain Care | | | | | | | | | | | |
| Rewopal LA 6 | 6.00 | 6.00 | 6.00 | 6.00 | 6.00 | 6.00 | 6.00 | 6.00 | | | |
| Marlipal 24/70 | | | | | | | | | | | |
| Mackamine LA | | | | | | | | | | | |
| Bio Saponins | | | | | | | | | | | |
| Steposol MET 10 U | | | | | | | | | | | |
| Betaine Coco Base | | | | | | | | | | | |
| **Anionics** | | | | | | | | | | | |
| SLES | 3.64 | 3.64 | 3.64 | 3.64 | 3.64 | 3.64 | 3.64 | 3.64 | 3.38 | 3.38 | 3.38 |
| Sulfated Castor Oil 75 WS | 3.00 | 3.00 | 3.00 | 3.00 | 3.00 | 3.00 | 3.00 | | 2.80 | 2.80 | 2.80 |
| Rewopol SBF A 30 B | 2.10 | 2.10 | 2.10 | 2.10 | 2.10 | 2.10 | 2.10 | 2.10 | 1.65 | 1.65 | 1.65 |
| Protelan LS9011 | | | | | | | | | | | |
| Greenbentin MLS/100 | | | | | | | | | | | |
| Eucarol Age SS | | | | | | | | | | | |
| TEA Laurylsulfat | | | | | | | | 2.40 | | | |
| **Emulsifieres/Stabiliser** | | | | | | | | | | | |
| Dermofeel Easymuls Plus | | | | | | | | | 1.5 | 1.5 | 1.5 |
| Dermosoft Octiol | | | | | | | | | | | |
| Versatil MPC | | | | | | | | | | | |
| Antilsoft SC | | | | | | | | | | | |
| Oxypon 401 | | | | | | | | | | | |
| Glycerin pflanzlich 99,5% | | | | | | | | | | | |
| Polyglycerin-6 | | | | | | | | | | | |
| Cetiol OE | | | | | | | | | | | |
| Glucopon 650 EC/HH | | | | | | | | | | | |
| Cosmacol ELI | | | | | | | | | | | |
| Tegosoft PC 31 | | 1.50 | 1.50 | 1.50 | 1.50 | 1.50 | 1.50 | 1.50 | | | |
| **Preservative** | | | | | | | | | | | |
| Organic acid | 0.60 | 0.60 | 0.60 | 0.60 | 0.60 | 0.60 | 0.60 | 0.60 | 0.60 | 0.60 | 0.60 |
| Organic acid | 0.30 | 0.30 | 0.30 | 0.30 | 0.30 | 0.30 | 0.30 | 0.30 | 0.30 | 0.30 | 0.30 |
| **Scrubber** | | | | | | | | | | | |
| Cornmeal | 7.50 | 7.50 | 7.50 | 7.50 | 7.50 | 7.50 | 7.50 | 7.50 | 7.50 | 7.50 | 7.50 |
| Olive Stone Granules | 4.00 | 4.00 | 4.00 | 4.00 | 4.00 | 4.00 | 4.00 | 4.00 | 4.00 | 4.00 | 4.00 |
| **Auxillaries** | | | | | | | | | | | |
| Fragrance | 0.20 | 0.20 | 0.20 | 0.20 | 0.20 | 0.20 | 0.20 | 0.20 | 0.20 | 0.20 | 0.20 |
| Palmera A1813 | 1.50 | 1.50 | 1.50 | 1.50 | 1.50 | 1.50 | 1.50 | | 1.50 | 1.50 | 1.50 |
| pH Adjuster | 0.45 | 0.45 | 0.45 | 0.45 | 0.45 | 0.45 | 0.45 | 0.45 | 0.45 | 0.45 | 0.45 |
| Mononatriumphosphat calc. | | | | | | | | 0.10 | | | |
| Sodiumchlorid solution 15% | | | | | | | | 0.50 | | | |
| **Performance** | | | | | | | | | | | |
| Cleansing effect | 95% | 95% | 95% | 95% | 95% | 95% | 95% | 95% | 74% | 74% | 74% |
| Foamability | 3.0 | 3.0 | 3.0 | 3.0 | 3.0 | 3.0 | 3.0 | 3.0 | 4.0 | 1.0 | 1.0 |
| Stability | - | - | - | - | - | - | - | - | + | - | + |

| Trade Name | O1.1 | O1.2 | O1.3 |
|---|---|---|---|
| Water | ad 100 | ad 100 | ad 100 |
| **Yield improvement** | | | |
| Keltrol CG-SFT | | | |
| Sucraclear HC-31 | | | |
| Jungbunzlauer FEDCS-PC | | | |
| Simulgel FL | | | |
| Exilva | | | |
| Aristoflex TAC | | | |
| Actigum VSX 20 | | | |
| Kelcogel CG-LA | | | |
| Rhodicare S | 0.45 | 0.40 | 0.35 |
| Walocel CRT 50.000 PA 07 | | | |
| **Nonionics** | | | |
| Gluco Tain Clear | 3.3 | 3.3 | 3.3 |
| Gluco Tain Care | | | |
| Rewopal LA 6 | | | |
| Marlipal 24/70 | | | |
| Mackamine LA | | | |
| Bio Saponins | | | |
| Steposol MET 10 U | | | |
| Betaine Coco Base | | | |
| **Anionics** | | | |
| SLES | 3.38 | 3.38 | 3.38 |
| Sulfated Castor Oil 75 WS | 2.80 | 2.80 | 2.80 |
| Rewopol SBF A 30 B | 1.65 | 1.65 | 1.65 |
| Protelan LS9011 | | | |
| Greenbentin MLS/100 | | | |
| Eucarol Age SS | | | |
| TEA Laurylsulfat | | | |
| **Emulsifieres/Stabiliser** | | | |
| Dermofeel Easymuls Plus | 1.5 | 1.5 | 1.5 |
| Dermosoft Octiol | | | |
| Versatil MPC | | | |
| Antilsoft SC | | | |
| Oxypon 401 | | | |
| Glycerin pflanzlich 99,5% | | | |
| Polyglycerin-6 | | | |
| Cetiol OE | | | |
| Glucopon 650 EC/HH | | | |
| Cosmacol ELI | | | |
| Tegosoft PC 31 | | | |
| **Preservative** | | | |
| Organic acid | 0.60 | 0.60 | 0.60 |
| Organic acid | 0.30 | 0.30 | 0.30 |
| **Scrubber** | | | |
| Cornmeal | 7.50 | 7.50 | 7.50 |
| Olive Stone Granules | 4.00 | 4.00 | 4.00 |
| **Auxillaries** | | | |
| Fragrance | 0.20 | 0.20 | 0.20 |
| Palmera A1813 | 1.50 | 1.50 | 1.50 |
| pH Adjuster | 0.45 | 0.45 | 0.45 |
| Mononatriumphosphat calc. | | | |
| Sodiumchlorid solution 15% | | | |
| **Performance** | | | |
| Cleansing effect | 74% | 74% | 74% |
| Foamability | 4.5 | 4.8 | 5.0 |
| Stability | + | + | + |

The data shows that using smooth flow xanthan gums and castor oil it is possible to produce stable foams

containing scrubbing agents. Using conventional xanthan gums, increased the viscosity to above optional levels unless concentration of the gum is controlled. Using surfactants, such as one or more fatty acid glucamides, such as capryloyl/caproyl methyl glucamide improves the product as does the addition of other surfactants such as SLS and DLS. Changing the xanthan gum for other polysaccharide gums, such as mixtures of other gums or gellan gums affects the properties of the final product away from optimum results.

Further work with conventional xanthan gums showed that 0.5% xanthan gum was able to be used to produce a foam. However, when stored for over 14 days the viscosity increased leading to reduced foam production. Viscosities of over 3500 cP decreased foam production.

## Claims

**1.** A hand pump foamer apparatus comprising a foamable skin and hand cleansing composition comprising the following components based on the total composition of the cleansing composition:

a) 0.5 to 10% by weight of sulphated castor oil;
b) 0.1 to 1% by weight of xanthan gum;
c) 0 to 5% by weight of at least one non-aqueous solvent, typically an organic solvent;
d) 1 to 30% by weight of at least on surfactant;
e) 0 to 3% by weight of at least one emulsifier;
f) 1 to 20% by weight of at least one scrubbing agent;
g) 0 to 2% by weight of at least one preservative;
h) 0 to 2% by weight of at least one pH modifier;
i) 0 to 10% by weight of auxiliaries and/or additive; and
j) water to make up 100% by weight.

wherein the surfactant (d) is selected from:

i) an amphoteric surfactant selected from any one or combination of betaines, acyl ethylene diamines, amino-acids derivatives or imidazolines; any one or combination of acylamphoacetate, acylamphodiacetate, acylamphodipropionate, sodium cocoglycinate, sodium alkyliminodipropionate, cocamidopropyl betaine or sodium cocoamphoacetate;
ii) a non-ionic surfactant selected from any one or combination of glucosides, ethoxylated fatty alcohols, ethoxylated fatty acids, saccharose esters, sorbitan esters, alkanolamides, glycerol alkyl esters or polyoxyethylene glycol alkylphenol esters;
iii) an anionic surfactant selected from any one or combination of lauryl sulphates, lauryl ether sulphates, sulphosuccinates, carboxylates, carboxylic acid esters, alkyl sulfate, alkyl and alkyl-aryl sulfonates, sulfosuccinates, isethionates, taurates acyl glutamates. sarcosinate, alkylpolyglucosides; or
iv) one or more fatty acid glucamides:

the emulsifier (e) is selected from:
PEG-18, castor dioleate, polyglycerin 4-caproate, d-n-octylether lauryl glucoside, caprylyl glycol, sorbitan sequicaprate, hydrogenated castor oil, PEG-10 glyceryl oleate and gemini surfactants and mixtures thereof, and no carboxymethylcellulose is incorporated.

**2.** A hand pump foamer apparatus according to claim 1, wherein the xanthan gum in the foamable skin and hand cleansing composition is smooth flow xanthan gum.

**3.** A hand pump foamer apparatus according to claims 1 or 2, wherein the foamable skin and hand cleansing composition has a viscosity of 200 to 5000 mPa.s.

**4.** A hand pump foamer apparatus according to claims 1 to 3, wherein the foamable skin and hand cleansing composition has a yield value of at least 0.0003N/cm$^2$ (30 dynes/cm$^2$).

**5.** A hand pump foamer apparatus according to claims 1 to 4 wherein the foamable skin and hand cleansing composition has a pH of below 5.5.

**6.** A hand pump foamer apparatus according to claims 1 to 5 wherein the foamable skin and hand cleansing composition

comprises 1.0 to 8% by weight, more typically 3 to 5% by weight of sulphated castor oil a).

**7.** A hand pump foamer apparatus according to claims 1 to 6 wherein the foamable skin and hand cleansing composition comprises 0.1 to 0.9% by weight of xanthan gum b).

**8.** A hand pump foamer apparatus according to claim 7, wherein the xanthan gum in the foamable skin and hand cleansing composition is conventional xanthan gum and the amount of xanthan gum is 0.1 to 0.5% by weight of the foamable skin and hand cleansing composition.

**9.** A hand pump foamer apparatus according to claims 1 to 8 wherein the foamable skin and hand cleansing composition comprises less than 3% by weight of non-aqueous solvent c).

**10.** A hand pump foamer apparatus according to claims 1 to 9 wherein the foamable skin and hand cleansing composition comprises 2 to 20%, typically 3 to 10% by weight of surfactant d).

**11.** A hand pump foamer apparatus according to claims 1 to 10 wherein in the foamable skin and hand cleansing composition, the surfactant d) is selected from one or more fatty acid glucamides, sodium laureth sulfate and disodium laureth sulfosuccinate and mixtures thereof.

**12.** A hand pump foamer apparatus according to claims 1 to 11, wherein the foamable skin and hand cleansing composition comprises 0.5 to 2.5% by weight, typically 1.0 to 2.0% by weight emulsifier e).

**13.** A hand pump foamer apparatus according to claims 1 to 12 wherein the foamable skin and hand cleansing composition comprises 5 to 15% by weight of scrubbing agent.

**14.** A hand pump foamer apparatus according to claims 1 to 13, wherein the foamable skin and hand cleansing composition comprises less than 0.5 wt % of an additional thickener.

**15.** A method of washing skin of a subject comprising expelling a composition from a foaming apparatus according to claim 1-14 to produce a foam and applying the foam to a portion of skin of the subject.

## Patentansprüche

**1.** Handpumpenschäumgerät, umfassend eine schäumbare Zusammensetzung zur Haut- und Handreinigung mit den folgenden Komponenten, jeweils bezogen auf die Gesamtzusammensetzung der Reinigungszusammensetzung:

a) 0,5 bis 10 Gew.-% sulfatiertes Rizinusöl;
b) 0,1 bis 1 Gew.-% Xanthan;
c) 0 bis 5 Gew.-% mindestens eines nichtwässrigen Lösungsmittels, typischerweise eines organischen Lösungsmittels;
d) 1 bis 30 Gew.-% mindestens eines Tensids;
e) 0 bis 3 Gew.-% mindestens eines Emulgators;
f) 1 bis 20 Gew.-% mindestens eines Waschmittels;
g) 0 bis 2 Gew.-% mindestens eines Konservierungsmittels;
h) 0 bis 2 Gew.-% mindestens eines pH-Modifizierungsmittels;
i) 0 bis 10 Gew.-% Hilfsstoffe und/oder Additive; und
j) Wasser zum Auffüllen auf 100 Gew.-%;

wobei das Tensid d) ausgewählt ist aus:

i) einem amphoteren Tensid, ausgewählt aus einem beliebigen oder einer Kombination von Betainen, Acylethylendiaminen, Aminosäurederivaten oder Imidazolinen; einem beliebigen oder einer Kombination von Acylamphoacetat, Acylamphodiacetat, Acylamphodipropionat, Natriumcocoglycinat, Natriumalkyliminodipropionat, Cocamidopropylbetain oder Natriumcocoamphoacetat;
ii) einem nichtionischen Tensid, ausgewählt aus einem beliebigen oder einer Kombination von Glucosiden, ethoxylierten Fettalkoholen, ethoxylierten Fettsäuren, Saccharoseestern, Sorbitanestern, Alkanolamiden, Glycerinalkylestern oder Polyoxyethylenglykolalkylphenolestern;

iii) einem anionischen Tensid, ausgewählt aus einem beliebigen oder einer Kombination von Laurylsulfaten, Laurylethersulfaten, Sulfosuccinaten, Carboxylaten, Carbonsäureestern, Alkylsulfat, Alkyl- und Alkylarylsulfonaten, Sulfosuccinaten, Isethionaten, Tauraten, Acylglutamaten, Sarkosinaten, Alkylpolyglucosiden; oder
iv) einem oder mehreren Fettsäureglucamiden;
wobei der Emulgator e) ausgewählt ist aus:
PEG-18, Rizinusdioleat, Polyglycerin-4-caproat, d-n-Octylether-Laurylglucosid, Caprylylglycol, Sorbitan-Sequicaprat, hydriertem Rizinusöl, PEG-10-Glyceryloleat und Geminitensiden, und Mischungen davon, und keine Carboxymethylcellulose beigegeben wird.

**2.** Handpumpenschäumgerät nach Anspruch 1, bei dem das Xanthan in der schäumbaren Haut- und Handreinigungszusammensetzung glattfließendes Xanthan ist.

**3.** Handpumpenschäumgerät nach Anspruch 1 oder 2, bei dem die Viskosität der schäumbaren Haut- und Handreinigungszusammensetzung im Bereich von 200 bis 5000 mPas liegt.

**4.** Handpumpenschäumgerät nach einem der Ansprüche 1 bis 3, bei dem die Fließgrenze der schäumbaren Haut- und Handreinigungszusammensetzung mindestens 0,0003 $N/cm^2$ (30 $dyn/cm^2$) beträgt.

**5.** Handpumpenschäumgerät nach einem der Ansprüche 1 bis 4, bei dem der pH-Wert der schäumbaren Haut- und Handreinigungszusammensetzung bei unter 5,5 liegt.

**6.** Handpumpenschäumgerät nach einem der Ansprüche 1 bis 5, bei dem die schäumbare Haut- und Handreinigungszusammensetzung 1,0 bis 8 Gew.-%, typischerweise 3 bis 5 Gew.-%, sulfatiertes Rizinusöl a) enthält.

**7.** Handpumpenschäumgerät nach einem der Ansprüche 1 bis 6, bei dem die schäumbare Haut- und Handreinigungszusammensetzung 0,1 bis 0,9 Gew.-% Xanthan b) enthält.

**8.** Handpumpenschäumgerät nach Anspruch 7, bei dem das Xanthan in der schäumbaren Haut- und Handreinigungszusammensetzung herkömmliches Xanthan ist und die Menge an Xanthan 0,1 bis 0,5 Gew.-% der schäumbaren Haut- und Handreinigungszusammensetzung beträgt.

**9.** Handpumpenschäumgerät nach einem der Ansprüche 1 bis 8, bei dem die schäumbare Haut- und Handreinigungszusammensetzung weniger als 3 Gew.-% des nichtwässrigen Lösungsmittels c) enthält.

**10.** Handpumpenschäumgerät nach einem der Ansprüche 1 bis 9, bei dem die schäumbare Haut- und Handreinigungszusammensetzung 2 bis 20 Gew.-%, typischerweise 3 bis 10 Gew.-%, an Tensid d) umfasst.

**11.** Handpumpenschäumgerät nach einem der Ansprüche 1 bis 10, bei dem in der schäumbaren Haut- und Handreinigungszusammensetzung das Tensid d) ausgewählt ist aus einem oder mehreren Fettsäureglucamiden, Natriumlaurethsulfat und Dinatriumlaurethsulfosuccinat und Mischungen davon.

**12.** Handpumpenschäumgerät nach einem der Ansprüche 1 bis 11, bei dem die schäumbare Haut- und Handreinigungszusammensetzung 0,5 bis 2,5 Gew.-%, typischerweise 1,0 bis 2,0 Gew.-%, an Emulgator e) umfasst.

**13.** Handpumpenschäumgerät nach einem der Ansprüche 1 bis 12, bei dem die schäumbare Haut- und Handreinigungszusammensetzung 5 bis 15 Gew.-% Waschmittel enthält.

**14.** Handpumpenschäumgerät nach einem der Ansprüche 1 bis 13, bei dem die schäumbare Haut- und Handreinigungszusammensetzung weniger als 0,5 Gew.-% eines zusätzlichen Verdickungsmittels enthält.

**15.** Verfahren zum Waschen der Haut eines Subjekts, umfassend das Ausstoßen einer Zusammensetzung aus einem Schäumgerät nach Anspruch 1 bis 14 zur Erzeugung eines Schaums und das Auftragen des Schaums auf einen Teil der Haut des Subjekts.

## Revendications

**1.** Appareil de production de mousse à pompe à main comprenant une composition nettoyante moussante pour la

peau et les mains comprenant les composants suivants sur la base de la composition totale de la composition nettoyante :

a) 0,5 à 10 % en poids d'huile de ricin sulfatée ;
b) 0,1 à 1 % en poids de gomme xanthane ;
c) 0 à 5 % en poids d'au moins un solvant non aqueux, typiquement un solvant organique ;
d) 1 à 30 % en poids d'au moins un tensioactif ;
e) 0 à 3 % en poids d'au moins un émulsifiant ;
f) 1 à 20 % en poids d'au moins un agent désincrustant ;
g) 0 à 2 % en poids d'au moins un conservateur ;
h) 0 à 2 % en poids d'au moins un modificateur de pH ;
i) 0 à 10 % en poids d'adjuvants et/ou d'additifs ; et
j) de l'eau pour compléter à 100 % en poids,

dans lequel le tensioactif (d) est choisi parmi :

i) un tensioactif amphotère choisi parmi l'un quelconque ou une combinaison de bétaïnes, acyléthylènediamines, dérivés d'acides aminés ou imidazolines ; l'un quelconque ou une combinaison d'acylamphoacétate, acylamphodiacétate, acylamphodipropionate, cocoglycinate de sodium, alkyliminodipropionate de sodium, cocamidopropylbétaïne ou cocoamphoacétate de sodium ;
ii) un tensioactif non ionique choisi parmi l'un quelconque ou une combinaison de glucosides, alcools gras éthoxylés, acides gras éthoxylés, esters de saccharose, esters de sorbitane, alcanolamides, esters d'alkyle de glycérol ou esters d'alkylphénol de polyoxyéthylène glycol ;
iii) un tensioactif anionique choisi parmi l'un quelconque ou une combinaison de sulfates de lauryle, sulfates d'éther de lauryle, sulfosuccinates, carboxylates, esters d'acide carboxylique, sulfate d'alkyle, sulfonates d'alkyle et d'alkylaryle, sulfosuccinates, iséthionates, taurates, glutamates d'acyle, sarcosinate, alkylpolyglucosides ; ou
iv) un ou plusieurs glucamides d'acide gras :
l'émulsifiant (e) est choisi parmi :
PEG-18, dioléate de ricin, 4-caproate de polyglycérine, laurylglucoside d'éther de d-n-octyle, caprylylglycol, séquicaprate de sorbitane, huile de ricin hydrogénée, PEG-10-oléate de glycéryle et des tensioactifs gémini et des mélanges de ceux-ci, et aucune carboxyméthylcellulose n'est incorporée.

**2.** Appareil de production de mousse à pompe à main selon la revendication 1, dans lequel la gomme xanthane dans la composition nettoyante moussante pour la peau et les mains est une gomme xanthane à écoulement fluide.

**3.** Appareil de production de mousse à pompe à main selon les revendications 1 ou 2, dans lequel la composition nettoyante moussante pour la peau et les mains a une viscosité de 200 à 5000 mPa.s.

**4.** Appareil de production de mousse à pompe à main selon les revendications 1 à 3, dans lequel la composition nettoyante moussante pour la peau et les mains a une valeur de rendement d'au moins 0,0003 $N/cm^2$ (30 $dynes/cm^2$).

**5.** Appareil de production de mousse à pompe à main selon les revendications 1 à 4 dans lequel la composition nettoyante moussante pour la peau et les mains a un pH inférieur à 5,5.

**6.** Appareil de production de mousse à pompe à main selon les revendications 1 à 5 dans lequel la composition nettoyante moussante pour la peau et les mains comprend 1,0 à 8 % en poids, plus typiquement 3 à 5 % en poids d'huile de ricin sulfatée a).

**7.** Appareil de production de mousse à pompe à main selon les revendications 1 à 6 dans lequel la composition nettoyante moussante pour la peau et les mains comprend 0,1 à 0,9 % en poids de gomme xanthane b).

**8.** Appareil de production de mousse à pompe à main selon la revendication 7, dans lequel la gomme xanthane dans la composition nettoyante moussante pour la peau et les mains est une gomme xanthane conventionnelle et la quantité de gomme xanthane est de 0,1 à 0,5 % en poids de la composition nettoyante moussante pour la peau et les mains.

**9.** Appareil de production de mousse à pompe à main selon les revendications 1 à 8 dans lequel la composition nettoyante moussante pour la peau et les mains comprend moins de 3 % en poids de solvant non aqueux c).

10. Appareil de production de mousse à pompe à main selon les revendications 1 à 9 dans lequel la composition nettoyante moussante pour la peau et les mains comprend 2 à 20 %, typiquement 3 à 10 % en poids de tensioactif d).

11. Appareil de production de mousse à pompe à main selon les revendications 1 à 10 dans lequel, dans la composition nettoyante moussante pour la peau et les mains, le tensioactif d) est choisi parmi un ou plusieurs glucamides d'acide gras, le lauréthsulfate de sodium et le lauréthsulfosuccinate disodique et des mélanges de ceux-ci.

12. Appareil de production de mousse à pompe à main selon les revendications 1 à 11, dans lequel la composition nettoyante moussante pour la peau et les mains comprend 0,5 à 2,5 % en poids, typiquement 1,0 à 2,0 % en poids d'émulsifiant e).

13. Appareil de production de mousse à pompe à main selon les revendications 1 à 12 dans lequel la composition nettoyante moussante pour la peau et les mains comprend 5 à 15 % en poids d'agent désincrustant.

14. Appareil de production de mousse à pompe à main selon les revendications 1 à 13, dans lequel la composition nettoyante moussante pour la peau et les mains comprend moins de 0,5 % en poids d'un épaississant supplémentaire.

15. Procédé de lavage de la peau d'un sujet comprenant l'expulsion d'une composition depuis un appareil de production de mousse selon la revendication 1 à 14 pour produire une mousse et appliquer la mousse sur une partie de la peau du sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014019944 A
- WO 2005107699 A
- US 201202027304 A
- EP 0005030 A
- US 4263399 A


### Non-patent literature cited in the description


- **S. K. HATE ; S.P. MOULIK.** *Current Science,* 2002, vol. 82 (9), 1101-1111